# EUROPEAN PATENT SPECIFICATION

(11) **EP 3 614 993 B1**
(45) Date of publication and mention of the grant of the patent: **17.02.2021**
(21) Application number: 18714761.6
(22) Date of filing: 29.03.2018
(51) Int. Cl.: A61K 8/27, A61Q 11/00, A61K 8/19

(54) **ORAL CARE COMPOSITION BASED ON ZINC OXIDE AND CALCIUM DIHYDROGEN PHOSPHATE**
MUNDPFLEGEZUSAMMENSETZUNG ENTHALTEN ZINKOXID UND KALCIUMDIHYDROGENPHOSPHAT
COMPOSITION DE PROTECTION ORALE COMPRENANT DE L'OXYDE DE ZINC DE L'HYDROGENOPHOSPHATE DE CALCIUM

(30) Priority: 27.04.2017 WO PCT/CN2017/082166; 22.05.2017 EP 17172173
(43) Date of publication of application: 04.03.2020
(73) Proprietor: Unilever N.V., 3013 AL Rotterdam (NL); Unilever PLC, London, Greater London EC4Y 0DY (GB)
(72) Inventor: LI, Xiaoke, Shanghai 200335 (CN); WANG, Jinfang, Shanghai 200335 (CN); XING, Huaiyong, Shanghai 200120 (CN)
(74) Representative: Tansley, Sally Elizabeth
(86) International application number: PCT/EP2018/058038
(87) International publication number: WO 2018/197148

(56) References cited:
- WO-A1-2011/123121
- WO-A1-2017/005431

## Description

### Technical Field of the Invention

The present invention relates to oral care compositions such as tooth pastes, powders, gums, mouthwashes and the like. In particular the present invention relates to an oral care composition comprising zinc oxide, calcium dihydrogen phosphate. The invention also relates to the use of such compositions for treating tooth hypersensitivity and/or benefiting teeth of an individual.

### Background of the Invention

Tooth hypersensitivity is a temporary induced pain sensation that affects up to 20% of the adult population. Hypersensitive teeth may be sensitive to temperature, pressure or chemical action.

The dentin of the tooth generally contains channels, called tubules, which provide for an osmotic flow between the inner pulp region of the tooth and the outer root surfaces. Tooth hypersensitivity may be related to the general increase in exposed root surfaces of teeth as a result of periodontal disease, toothbrush abrasion, or cyclic loading fatigue of the thin enamel near the dentin-enamel junction. When root surfaces are exposed, dentinal tubules are also exposed.

The currently accepted theory for tooth hypersensitivity is the hydrodynamic theory, based on the belief that open exposed dentinal tubules allow fluid flow through the tubules. This flow excites the nerve endings in the dental pulp. Clinical replica of sensitive teeth viewed in a SEM (scanning electron microscopy) reveal varying numbers of open or partially occluded dentinal tubules.

There are different approaches to treat tooth hypersensitivity. One approach is to reduce the excitability of the nerve in a sensitive tooth by using "nerve-depolarising agents" comprising strontium ions, potassium salts such as potassium nitrate, potassium bicarbonate, potassium chloride and the like. These nerve-depolarising agents function by interfering with neural transduction of the pain stimulus to make the nerve less sensitive.

Another approach is to use "tubule blocking agents" that fully or partially occlude tubules such as polystyrene beads, apatite, polyacrylic acid, mineral hectorite clay and the like. These tubule blocking agents function by physically blocking the exposed ends of the dentinal tubules, thereby reducing dentinal fluid movement and reducing the irritation associated with the shear stress described by the hydrodynamic theory.

There is a continuing need for treating tooth hypersensitivity with a more efficient approach. The present inventors have now found unexpectedly that an oral care composition comprising zinc oxide, calcium dihydrogen phosphate provides excellent tubule blockage efficacy to reduce tooth sensitivity. In addition, the deposition of zinc oxide on the tooth surfaces may release antibacterial metal zinc into saliva and/or areas of the oral cavity and provide a long lasting *in situ* antibacterial benefit. Furthermore, it has also been found unexpectedly that such a composition can enhance the delivery and deposition of benefit agents on tooth surfaces, thus improving the delivery efficiency of those benefit agents to provide enhanced tooth benefits.

### Additional Information

GB 1,049,036 (Biorex Laboratories Ltd) discloses a dental cement comprising zinc oxide, a hydrogenated rosin and/or an ester thereof an o-ethoxy-benzoic acid.

US 3,751,391 (National Research Development Corp) discloses a process for the preparation of a surgical cement by mixing a surgical grade metal oxide powder with a water-soluble polymer of acrylic acid and water to give a plastic mass that rapidly hardens.

WO 2011/123121 A1 (Colgate) discloses oral care compositions comprising an orally acceptable vehicle, metal oxide particles having an average particle size of no greater than a dentin tubule and a polymeric adherent material for adhering the metal oxide particles in the dentin tubule.

WO 2017/005431 A1 (Unilever) discloses an oral care composition comprising calcium silicate, a deposition aid selected from calcium sulfate hemihydrate, calcium dihydrogen phosphate or mixtures thereof and a physiologically acceptable carrier, wherein the calcium silicate and the deposition aid is present in a weight ratio from 20:1 to 1:5.

The additional information above does not describe an oral care composition comprising zinc oxide and calcium dihydrogen phosphate, wherein the zinc oxide and the calcium dihydrogen phosphate are present in a weight ratio from 10:1 to 1:5, and particularly such oral care composition can treat tooth hypersensitivity and/or enhance the deposition of benefit agents on tooth surfaces.

### Tests and Definitions

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purpose of the present invention means a paste or gel dentifrice for use with a toothbrush. Especially preferred are tooth pastes suitable for cleaning teeth by brushing for about two minutes.

### Mouth Wash

"Mouth wash" for the purpose of the present invention means liquid dentifrice for use in rinsing the mouth. Especially preferred are mouth washes suitable for rinsing the mouth by swishing and/or gargling for about half a minute before expectorating.

### Particle Size

"Particle size" for the purpose of the present invention means particle diameter unless otherwise stated. Diameter is meant to mean the largest measurable distance on a particle in the event a well-defined sphere is not generated. Particle size can be measured, for example, by dynamic light scattering (DLS).

### Composite Particle

"Composite particle" for the purpose of the present invention means a particle comprising a first component core and a second component coating wherein the core and coating are composed of different materials.

### Refractive Index

Refractive index is quoted at a temperature of 25°C and a wavelength of 589 nm.

### pH

pH is quoted at atmospheric pressure and a temperature of 25°C. When referring to the pH of an oral care composition, this means the pH measured when 5 parts by weight of the composition is uniformly dispersed and/or dissolved in 20 parts by weight pure water at 25°C. In particular the pH may be measured by manually mixing 5 g oral care composition with 20 mL water for 30 s, then immediately testing the pH with indicator or a pH meter.

### Substantially Free

"Substantially free of" for the purpose of the present invention means less than 1.5%, and preferably less than 1.0%, and more preferably less than 0.75% and more preferably still less than 0.5%, and even more preferably less than 0.1% and most preferably from 0.0 to 0.01% by weight, based on total weight of the oral care composition, including all ranges subsumed therein.

### Viscosity

Viscosity of a tooth paste is the value taken at room temperature (25°C) with a Brookfield Viscometer, Spindle No.4 and at a speed of 5 rpm. Values are quoted in centipoises (cP=mPa.s) unless otherwise specified.

### Remineralization

"Remineralization" for the purpose of the present invention means *in situ* (i.e. in the oral cavity) generation of calcium phosphate on teeth (including layers on teeth from 10 nm to 20 microns, and preferably from 75 nm to 10 microns, and most preferably, from 150 nm to 5 microns thick including all ranges subsumed therein) to reduce the likelihood of tooth sensitivity, tooth decay, regenerate enamel and/or improve the appearance of teeth by whitening through the generation of such new calcium phosphate.

### Miscellaneous

Except in the examples, or where otherwise explicitly indicated, all numbers in this description indicating amounts of material or conditions of reaction, physical properties of materials and/or use may optionally be understood as modified by the word "about". All amounts are by weight of the final oral care composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Summary of the Invention

In a first aspect, the present invention is directed to an oral care composition comprising:
a) zinc oxide; and
b) calcium dihydrogen phosphate;
wherein the zinc oxide and the calcium dihydrogen phosphate are present in a weight ratio from 10:1 to 1:5.

In a second aspect, the present invention is directed to a packaged oral care product comprising the oral care composition of the first aspect of this invention.

In a third aspect, the present invention is directed to a method of benefiting teeth of an individual comprising the step of applying the oral care composition of any embodiment of the first aspect to at least one surface of the teeth of the individual. Particularly, the present invention is directed to a method of reducing sensitivity and/or remineralizing and/or whitening of teeth of an individual.

All other aspects of the present invention will more readily become apparent upon considering the detailed description and examples which follow.

### Detailed Description

Now it has been found that an oral care composition comprising zinc oxide and calcium dihydrogen phosphate provides excellent tubule blockage efficacy to reduce tooth sensitivity. In addition, the deposition of zinc oxide on the tooth surfaces may release antibacterial metal zinc into saliva and/or areas of the oral cavity and provide a long lasting *in situ* antibacterial benefit. Furthermore, it has also been found unexpectedly that such a composition can enhance the delivery and deposition of benefit agents on tooth surfaces, thus improving the delivery efficiency of those benefit agents to provide enhanced tooth benefits. Delivery efficiency, as used herein, means the ability to deliver and deposit benefit agents to tooth surfaces of an individual.

Zinc oxide is an inorganic compound with the formula ZnO. Preferably, the zinc oxide according to the present invention is particulate that can be of different sizes and shapes. The particles may be of spherical, platelet or irregular shape form. Particle size, as used herein, refers to particle diameter unless otherwise stated. Diameter is meant to mean the largest measurable distance on a particle in the event a well-defined sphere is not generated. Particle size can be measured, for example, by dynamic light scattering (DLS). The diameter of the zinc oxide particles is often from 10 nm to less than 50 microns, preferably from 50 nm to less than 10 microns, more preferably from 75 nm to 5 microns and most preferably from 100 nm to 1 micron, including all ranges subsumed therein. Suitable zinc oxide particles are made commercially available, for example, from suppliers like Aladdin.

Alternatively or additionally, the oral care composition according to the present invention may comprise other metal salts or oxides. Illustrative yet non-limiting examples of the types of metal salts or oxides that may be used in this invention include, for example, calcium fluoride, oxides of magnesium, bismuth, calcium, copper, strontium, barium and silver, mixtures thereof or the like.

Typically, the oral care composition of the present invention comprises from 0.1 to 35%, and more preferably from 0.5 to 20%, and most preferably, from 1 to 10% by weight of the zinc oxide, based on the total weight of the oral care composition and including all ranges subsumed therein.

Calcium dihydrogen phosphate is also called monocalcium phosphate anhydrous. It is unexpectedly found that calcium dihydrogen phosphate which acts as tubule blockage enhancer in this invention is biocompatible, and undergoes rapid reaction in water and complete resorption onto the dentine and/or enamel of teeth. The interaction between zinc oxide and calcium dihydrogen phosphate helps their deposition onto dentine and/or into dentinal tubules to induce *in situ* generation of zinc calcium phosphate that occludes the dentinal tubules and/or their exposed open ends. When benefit agents are present in the oral care composition, the generation of zinc calcium phosphate around the benefit agents further helps the retention of those benefit agents on tooth surfaces by enhancing their resistance to shear force. Suitable calcium dihydrogen phosphate are made commercially available, for example, from suppliers like Lianyungang Debang Fine Chemical Co., Ltd.

Alternatively, or additionally, the oral care composition according to the present invention may comprise other tubule blockage enhancer. Illustrative yet non-limiting examples of the types of tubule blockage enhancer that may be used in this invention include, for example, calcium sulfate hemihydrates, calcium aluminate, mixtures thereof or the like.

The oral care composition typically comprises from 0.01 to 20% by weight of the calcium dihydrogen phosphate, more preferably from 0.05 to 10%, more preferably still from 0.1 to 8%, and most preferably from 0.5 to 5%, based on the total weight of the oral care composition and including all ranges subsumed therein.

The oral care composition comprises the zinc oxide and the calcium dihydrogen phosphate in a weight ratio from 10:1 to 1:5, preferably from 5:1 to 1:3, and more preferably from 3:1 to 1:2.

The composition of the present invention is an oral care composition and typically comprises a physiologically acceptable carrier. The carrier preferably comprises at least surfactant, thickener, humectant or a combination thereof.

Preferably the oral care composition comprises a surfactant. Preferably the composition comprises at least 0.01% surfactant by weight of the composition, more preferably at least 0.1% and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate, sodium coco sulfate, cocamidopropyl betaine, sodium methyl cocoyl taurate or mixtures thereof.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, xanthan gum and/or sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another especially preferred embodiment, the thickener is xanthan gum.

Thickener typically makes up from 0.01 to about 10%, more preferably from 0.1 to 9%, and most preferably, from 0.1 to 5% by weight of the oral care composition, based on total weight of the composition and including all ranges subsumed therein.

When the oral care composition of this invention is a toothpaste or gel, the same typically has a viscosity from about 30,000 to 180,000 centipoise, and preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. More preferably, the carrier humectant makes up from 25 to 80%, and most preferably, from 30 to 60% by weight of the composition, based on total weight of the composition and including all ranges subsumed therein.

The oral care composition may further comprise benefit agents that are typically delivered to human teeth and/or the oral cavity including the gums to enhance or improve a characteristic of those dental tissues. The only limitation with respect to the benefit agents that may be used in this invention is that the same is suitable for use in the mouth. The benefit agent is present in the oral care composition in addition to the zinc oxide and the calcium dihydrogen phosphate that are included in the composition.

Typically the benefit agent is selected from optical agents, biomineralization agents, antibacterial agents, gum health agents, desensitizing agents, anti-calculus agents, freshness agents or mixtures thereof. Preferably, the benefit agent is selected from optical agents, biomineralization agents, antibacterial agents, gum health agents, freshness agents or mixtures thereof.

For example, optical agents such as coloring agents like whitening agents and pigments. Preferably, the pigment, when used, is violet or blue having a hue angle, h, in the CIELAB system of from 220 to 320 degrees. These pigments may be selected from one or more of those listed in the Colour Index International, listed as pigment blue 1 through to pigment blue 83, and pigment violet 1 through to pigment violet 56. In another preferred embodiment, the optical agents may be selected from one or more of mica, interference mica, boron nitride, poly(methyl methacrylate) flake, composite microspheres, titanium dioxide coated glass flake, inverse opal, cholesteric liquid crystal, photonic sphere, hollow sphere and zinc oxide. Biomineralization agents for tooth enamel remineralization may be selected from one or more of fluoride sources, biomolecules, proteinaceous materials, amorphous calcium phosphate, α-tricalcium phosphate, β-tricalcium phosphate; calcium carbonate, calcium deficient hydroxyapatite Ca_{10-X}(HPO₄)_{X}(PO₄)_{6-X}(OH)_{2-X}, 0 ≤ x < 1), dicalcium phosphate (CaHPO₄), dicalcium phosphate dihydrate (CaHPO₄·2H₂O), hydroxyapatite (Ca₁₀(PO₄)₆(OH)₂), monocalcium phosphate monohydrate (Ca(H₂PO₄)₂·H₂O), octacalcium phosphate (Ca₈H₂(PO₄)₆·5H₂O) and tetracalcium phosphate (Ca₄(PO₄)₂O). Antibacterial agents may be selected from one or more of metal salts where the metal is selected from zinc, copper, silver or a mixture thereof, triclosan, triclosan monophosphate, triclocarban, curcumin, quaternary ammonium compounds, bisbiguanides and long chain tertiary amines, preferably zinc salts including zinc oxide, zinc chloride, zinc acetate, zinc ascorbate, zinc sulphate, zinc nitrate, zinc citrate, zinc lactate, zinc peroxide, zinc fluoride, zinc ammonium sulfate, zinc bromide, zinc iodide, zinc gluconate, zinc tartarate, zinc succinate, zinc formate, zinc phenol sulfonate, zinc salicylate, zinc glycerophosphate or a mixture thereof. Gum health agents may be selected from one or more of anti-inflammatory agents, plaque buffers, biomolecules, proteinaceous materials, vitamin, plant extracts and curcumin. Freshness agents may be flavors selected from one or more of peppermint, spearmint, menthol, flora oil, clove oil and citrus oil.

The benefit agent is preferably particulate as this allows for maximum surface area for contact with dental tissue.

In a preferred embodiment, the benefit agent is a particulate whitening agent for tooth whitening.

Typically, the particulate whitening agent comprises a material suitable to physically and immediately improve characteristics of teeth and especially whiten teeth. In order to provide excellent whitening effect, the material is preferred to have a high refractive index of at least 1.9, more preferably at least 2.0, even more preferably at least 2.2, even more preferably still at least 2.4 and most preferably at least 2.5. The maximum refractive index of the material is not particularly limited but preferably up to 4.0. Preferably, the material has a refractive index ranging from 1.9 to 4.0.

Particularly suitable materials are metal compounds and preferred are compounds where the metal is selected from zinc (Zn), titanium (Ti), zirconium (Zr) or a combination thereof. Preferably, the metal compound is (or at least comprises) a metal oxide such as titanium dioxide (TiO₂), zinc oxide (ZnO), zirconium dioxide (ZrO₂) or a combination thereof. In addition, the particulate whitening agent can also comprise non-metal oxides such as strontium titanate and zinc sulfide.

In a preferred embodiment, the particulate whitening agent comprises metal oxides, non-metal oxides or a combination thereof in an amount of at least 50% by weight of the whitening agent and more preferably at least 70%, more preferably still from 80 to 100% and most preferably from 85 to 95%. In an especially preferred embodiment, the particulate whitening agent is at least 50% by weight titanium dioxide, and most preferably, from 60 to 100% by weight titanium dioxide, based on total weight of the whitening agent and including all ranges subsumed therein. In another especially preferred embodiment, the particulate whitening agents are slightly soluble or insoluble in water, but most preferably, insoluble in water.

In a preferred embodiment, the particulate whitening agents are composite particles. The refractive index of a composite particle comprising more than one material can be calculated based on the refractive indices and volume fractions of the constituents using effective medium theory, as is described for example in WO 2009/023353.

The composite particle comprises a first component core and a second component coating. Typically, the core of the composite particle comprises a material suitable to physically and immediately improve characteristics of teeth and especially whiten teeth. In order to provide excellent whitening effect, the material is preferred to have a high refractive index of at least 1.9, more preferably at least 2.0, even more preferably at least 2.2, even more preferably still at least 2.4 and most preferably at least 2.5. The maximum refractive index of the material is not particularly limited but preferably up to 4.0. Preferably, the material has a refractive index ranging from 1.9 to 4.0.

Particular suitable materials are metal compounds and preferred are compounds where the metal is selected from zinc (Zn), titanium (Ti), zirconium (Zr) or a combination thereof. Preferably, the metal compound is (or at least comprises) a metal oxide such as titanium dioxide (TiO₂), zinc oxide (ZnO), zirconium dioxide (ZrO₂) or a combination thereof. In addition, the core of the composite particle can also comprise non-metal oxides such as strontium titanate and zinc sulfide.

The core of the composite particle typically makes up from 3 to 98%, and preferably from 6 to 65%, and most preferably from 10 to 55% by weight of the composite particle, based on total weight of the composite particle and including all ranges subsumed therein. In a preferred embodiment, the core comprises metal oxides, non-metal oxides or a combination thereof in an amount of at least 50% by weight of the core and more preferably at least 70%, more preferably still from 80 to 100% and most preferably from 85 to 95%. In an especially preferred embodiment, the core is at least 50% by weight titanium dioxide, and most preferably, from 60 to 100% by weight titanium dioxide, based on total weight of the first component core.

The second component coating comprises material suitable to adhere to tooth enamel, dentin or both. Typically, the coating material comprises the element calcium, and optionally, other metals like potassium, sodium, aluminium, magnesium as well as mixtures thereof whereby such optional metals are provided as, for example, sulphates, lactates, oxides, carbonates or silicates. Optionally, the coating material may be aluminium oxide or silica. In a preferred embodiment, the coating material is suitable to provide a biological or chemical improvement to teeth which is long term (e.g., results in hydroxyapatite formation). Preferably, the coating employed comprises at least 50% by weight elemental calcium, and most preferably, at least 65% by weight elemental calcium based on total weight of metal in the coating. In an especially preferred embodiment, the metal in the coating is from 80 to 100% by weight of elemental calcium, based on total weight of metal in the second component coating and including all ranges subsumed therein. In another especially preferred embodiment, the core and the coating are slightly soluble or insoluble in water, but most preferably, insoluble in water.

In an especially desired embodiment, the second component coating can comprise, for example, calcium phosphate, calcium gluconate, calcium oxide, calcium lactate, calcium carbonate, calcium hydroxide, calcium sulphate, calcium carboxymethyl cellulose, calcium alginate, calcium salts of citric acid, calcium silicate, mixture thereof or the like. In another desired embodiment, the calcium source in the coating comprises calcium silicate.

In yet another preferred embodiment, the coating can comprise the element calcium which originates from insoluble calcium silicate, present as the composite material calcium oxide-silica (CaO-SiO₂) as described in international patent applications published as WO 2008/015117 and WO 2008/068248.

When a calcium silicate composite material is employed as coating, the ratio of calcium to silicon (Ca:Si) may be from 1:10 to 3:1. The Ca:Si ratio is preferably from 1:5 to 3:1, and more preferably, from 1:3 to 3:1, and most preferably, from about 1:2 to 3:1. The calcium silicate may comprise mono-calcium silicate, bi-calcium silicate, or tri-calcium silicate whereby ratios of calcium to silicon (Ca:Si) should be understood to be atom ratios.

Usually, at least 30% of the outer surface area of the first component core is coated with the second component coating, preferably at least 50% of the core is coated with the coating, most preferably, from 70 to 100% of the outer surface area of the first component core is coated with the second component coating.

In an especially preferred embodiment, the particulate whitening agent is titanium dioxide coated with calcium silicate.

The particulate whitening agent according to the present invention can be of different sizes and shapes. The particles may be of spherical, platelet or irregular shape form. The diameter of the particulate whitening agent is often from 10 nm to less than 50 microns, and preferably, from 75 nm to less than 10 microns. In an especially preferred embodiment, the diameter of particles is from 100 nm to 5 microns, including all ranges subsumed therein. For composite particles, in a preferred embodiment, at least 40%, and preferably, at least 60%, and most preferably, from 75 to 99.5% of the diameter of the composite particle is the core, including all ranges subsumed therein.

In another preferred embodiment, the benefit agent is a biomineralization agent for tooth enamel remineralization selected from one or more of fluoride sources, biomolecules, proteinaceous materials, amorphous calcium phosphate, α-tricalcium phosphate, β-tricalcium phosphate; calcium carbonate, calcium deficient hydroxyapatite Ca_{10-X}(HPO₄)_{X}(PO₄)_{6-X}(OH)_{2-X}, 0 ≤ x < 1), dicalcium phosphate (CaHPO₄), dicalcium phosphate dihydrate (CaHPO₄·2H₂O), hydroxyapatite (Ca₁₀(PO₄)₆(OH)₂), monocalcium phosphate monohydrate (Ca(H₂PO₄)₂·H₂O), octacalcium phosphate (Ca₈H₂(PO₄)₆·5H₂O) and tetracalcium phosphate (Ca₄(PO₄)₂O). Preferably, the biomineralization agent is hydroxyapatite.

In an especially preferred embodiment, the benefit agent comprises or is a combination of biomineralization agents and optical agents. Preferably, the biomineralization agent is hydroxyapatite and the optical agent is a particulate whitening agent. Without wishing to be bound by theory, the present inventors believe that the presence of biomineralization agents helps the deposition and retention of optical agents on tooth surfaces to provide enhanced benefits such as tooth whitening.

The oral care composition of the present invention may comprise a single benefit agent or a mixture of two or more benefit agents. Typically, the benefit agent is present in an amount from 0.25 to 60%, and more preferably, from 0.5 to 40%, and most preferably, from 1 to 30% by total weight of the oral care composition and including all ranges subsumed therein.

When the benefit agent is incorporated into the oral care composition, the weight ratio of zinc oxide to benefit agent may range from 1:30 to 20:1, preferably from 1:20 to 10:1, most preferably from 1:15 to 5:1.

The oral care composition of the present invention may contain a variety of other ingredients which are common in the art to enhance physical properties and performance. These ingredients include preservatives, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

The oral care composition of this invention can be used in a method of benefiting teeth of an individual comprising applying in the composition to at least one surface of the teeth of an individual, said benefits includes reduced sensitivity, remineralization, whitening and combinations thereof. The oral care composition of this invention may additionally or alternatively be for use as a medicament and/or used in the manufacture of a medicament for providing an oral care benefit as described herein, such as for reducing sensitivity of and/or remineralizing and/or whitening the teeth of an individual. Alternatively and preferably, the use is non-therapeutic.

Preferably, the oral care composition is substantially free of water to prevent the premature reaction between the zinc oxide and calcium dihydrogen phosphate. In a preferred embodiment, the oral care composition is a monophase anhydrous composition.

In another preferred embodiment, the oral care composition is a dual-phase composition comprising a first phase and a second phase, wherein the zinc oxide is present in the first phase and the calcium dihydrogen phosphate is present in the second phase. When a benefit agent is incorporated into the oral care composition, the benefit agent can be present in either of the two phases. The delivery of the two independent phases to the teeth may be simultaneous or sequential. In a preferred embodiment, the phases are delivered simultaneously.

Typically, the dual-phase composition is delivered by a dual-tube having a first compartment for the first phase and a second compartment for the second phase, which allows for co-extrusion of the two phases.

In a preferred embodiment, such a dual-tube has one of the compartments surrounding the other. In such embodiments, one phase is present as a sheath, surrounding the other phase in the core. In an especially preferred embodiment, the core is the first phase and the sheath is the second phase.

In another preferred embodiment, such a dual-tube has the two compartments side by side within the same tube. In such embodiments, the two phases are extruded from the tube as one, such extrusion being termed "contact extrusion". A pump head may be used in such a dual-tube for squeezing the two phases from the tube as one.

The dual-phase oral care composition may be a gel composition that comprises two independent gel phases, one is the first phase and the other one is the second phase. The means of delivery may involve a cotton rod, or a tray, onto which the first phase and the second phase are applied, prior to the tray being placed in contact with the teeth. Typically, the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area.

The composition can be effective even when used in an individual's daily oral hygiene routine. For example, the composition may be brushed onto the teeth. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day. When the oral care composition is a dual-phase composition, the two phases of the composition are mixed during application. The mixed phases are typically left on the teeth for from 3 minutes to 10 hours, more preferably from 3 minutes to 8 hours. The application may be carried out daily.

The following examples are provided to facilitate an understanding of the present invention.

### Examples

### Example 1

This example demonstrates the improved blockage of dentinal tubules by using zinc oxide in combination with calcium dihydrogen phosphate. All ingredients are expressed by weight percent of the total formulation, and as level of active ingredient.

**TABLE 1**

| Ingredient | Samples | | |
|---|---|---|---|
| | **1** | **2** | **3** |
| Zinc oxide | 2.0 | -- | 2.0 |
| Calcium dihydrogen phosphate | -- | 1.0 | 1.0 |
| Glycerin | 98.0 | 99.0 | 97.0 |

### Methods

### Samples Preparation

To evaluate blockage efficacy of dentinal tubules, the test sample was mixed with water at a ratio of 4g to 8mL water to make a slurry.

Human dentine discs were eroded by 37% phosphoric acid for 1 min, then they were treated with different slurries via brushing following the same protocol. Six human dentine discs were separated into three groups (n=2). The dentine discs were brushed with the slurry under a tooth brushing machine equipped with toothbrushes. The load of the tooth brushing was 170 g +/-5 g and the automatic brushing operated at a speed of 150 rpm. After brushing for 1 min, the dentine discs were soaked in toothpaste slurry for 1 min. Then the dentine discs were rinsed with distilled water and placed in simulated oral fluid (SOF) under the condition of a shaking water bath at 37°C and 60.0 rpm. After soaking for about 3 to 4 hours, the dentine discs were brushed with the slurry by machine using the same procedure as in the first step. The brushing was repeated three times for one day, then the dentine discs were kept in SOF overnight(>12 hours) in a shaking water bath at 37°C to mimic oral environment. The dentine samples were brushed for 14 times.

Simulated oral fluid was made by combining the ingredients in Table 2:

**TABLE 2**

| Ingredient | Amount/g |
|---|---|
| NaCl | 16.07 |
| NaHCO₃ | 0.7 |
| KCl | 0.448 |
| K₂HPO₄*3H₂O | 3.27 |
| MgCl₂*6H₂O | 0.0622 |
| 1M HCl | 40 mL |
| CaCl₂ | 0.1998 |
| Na₂SO₄ | 0.1434 |
| Buffer | Adjust pH to 7.0 |
| Water | Balance to 2 L |

### Scoring Standard for Tubules Blockage

Regardless of the original shape of the dentine discs, a square (with a size of 4mm x 4mm) is selected and one image is captured under 50x magnification. Within this square, five spots (each with a size of 150 µm x 150 µm, one in the middle, and one in every corner) are selected and observed under 1000x magnification. The blockage of tubules are accessed following the standards described in Table 3. The measurement is carried out for the two dentine discs of each test group.

**TABLE 3**

| **Score** | **Tubules Blockage** |
|---|---|
| 0 | All dentinal tubules are open. |
| 1 | <20% of dentinal tubules are fully blocked. |
| 2 | 20 to 50% of dentinal tubules are fully blocked. |
| 3 | 50 to 80% of dentinal tubules are fully blocked. |
| 4 | 80-100% of dentinal tubules are fully blocked. |
| 5 | All dentinal tubules are fully blocked. |

### Results

After 14 brushings, SEM (Scanning Electron Microscopy) images of the dentine discs were taken. The images were analyzed and scored. The results are summarized in Table 4 (error represents standard deviation for duplicate measurements).

**TABLE 4**

| Tubules Blockage Score | Samples | | |
|---|---|---|---|
| | **1** | **2** | **3** |
| 14 brushings^{a} | 1.3^{A} ± 0.48 | 1.1^{A} ± 0.32 | 4.6^{B} ± 0.52 |

| | | | |
|---|---|---|---|
| a. Values with different letter are significantly different (p<0.01). | | | |

It showed that after 14 brushings, Sample 3 comprising a combination of zinc oxide and calcium dihydrogen phosphate showed significantly better tubule blockage efficacy compared to Samples 1 and 2 comprising only zinc oxide or calcium dihydrogen phosphate respectively. SEM images clearly showed that all the dentinal tubules were extensively blocked after treated with Sample 3, while for Samples 1 or 2, lots of tubules were still open.

### Example 2

This example demonstrates the improved deposition of whitening particles on tooth surfaces by using zinc oxide in combination with calcium dihydrogen phosphate. All ingredients are expressed by weight percent of the total formulation, and as level of active ingredient.

**TABLE 5**

| Ingredient | Samples | | | |
|---|---|---|---|---|
| | **4** | **5** | **6** | **7** |
| Zinc oxide | -- | 2.0 | -- | 2.0 |
| Calcium dihydrogen phosphate | -- | -- | 1.0 | 1.0 |
| Titanium dioxide coated with calcium silicate^{b} | 15.0 | 15.0 | 15.0 | 15.0 |
| Glycerin | 85.0 | 83.0 | 84.0 | 82.0 |

| | | | | |
|---|---|---|---|---|
| b. Commercially available titanium dioxide coated with calcium silicate from KOBO Products Inc. | | | | |

### Methods

To evaluate tooth whitening efficacy of the samples, the following in vitro test was performed. Changes in whiteness was measured using a Konica Minolta-2600D colorimeter. The WIO index is an index which has been optimized specifically for the evaluation of whiteness in teeth (as described in Journal of Dentistry, volume 36, Supplement 1, 2008, pages 2 to 7).

The test sample was pre-mixed using vortex and then added onto the enamel surface, water was added at the beginning of brushing. The test sample was added at a ratio of 4g to 8mL water.

The bovine enamel blocks were treated with different samples via brushing following the same protocol. The enamel blocks were brushed with the samples under a tooth brushing machine equipped with toothbrushes. The load of the tooth brushing was 170g +/-5 g and the automatic brushing operated at a speed of 150 rpm. After brushing for 1 min, the enamel blocks were rinsed with distilled water and soaked in SOF under the condition of a shaking water bath at 37°C and 60.0 rpm. After soaking for 3 to 4 hours, the enamel blocks were brushed with the samples by machine using the same procedure as in the first step. The brushing was repeated three times for one day, then the enamel blocks were kept in SOF overnight (>12 hours) in a shaking water bath at 37°C to mimic oral environment. After overnight incubation in SOF, the enamel blocks were brushed with water by machine using the same procedure as brushing with samples. These steps are considered as a whole treatment cycle. The enamel blocks were treated for one and three cycles. The changes in WIO values (ΔWIO) from baseline were calculated and statistically analyzed.

### Results

The results of tooth whitening after one cycle and three cycles are summarized in table 6 (error represents standard deviation for duplicate measurements).

**TABLE 6**

| Change in WIO | Samples | | | |
|---|---|---|---|---|
| | **4** | **5** | **6** | **7** |
| One cycle | 1.70 ± 1.08 | 2.41 ± 2.09 | 0.93 ± 1.03 | 2.51 ± 1.40 |
| Three cycles^{c} | 0.98^{A} ± 1.57 | 1.11^{A} ± 1.84 | 1.22^{A} ± 2.29 | 4.21 ^{B} ± 1.76 |

| | | | | |
|---|---|---|---|---|
| c. Values with different letter are significantly different (p<0.05). | | | | |

Sample 4 was a comparative example comprising particles only. Samples 5 and 6, which comprised zinc oxide or calcium dihydrogen phosphate respectively in addition to the particles, showed comparable tooth whitening efficacy to Sample 4 after one cycle and three cycles of treatment. Sample 7 comprising a combination of zinc oxide and calcium dihydrogen phosphate showed significantly better tooth whitening efficacy than other samples.

### Examples 3

This example demonstrates the improved deposition of hydroxyapatite particles on tooth surfaces by using zinc oxide in combination with calcium dihydrogen phosphate. All ingredients are expressed by weight percent of the total formulation, and as level of active ingredient.

**TABLE 7**

| Ingredient | Samples | | | |
|---|---|---|---|---|
| | **8** | **9** | **10** | **11** |
| Zinc oxide | -- | 2.0 | 2.0 | 2.0 |
| Calcium dihydrogen phosphate | -- | 1.0 | 1.0 | 1.0 |
| Hydroxyapatite | 10.0 | 10.0 | -- | 10.0 |
| Titanium dioxide | -- | -- | 15.0 | 15.0 |
| Glycerin | 90.0 | 87.0 | 82.0 | 72.0 |

### Methods

The test sample was pre-mixed using vortex and then added onto the enamel surface, water was added at the beginning of brushing. The test sample was added at a ratio of 4g to 8mL water.

The bovine enamel blocks were treated with different samples via brushing following the same protocol. The enamel blocks were brushed with the samples under a tooth brushing machine equipped with toothbrushes. The load of the tooth brushing was 170g +/-5 g and the automatic brushing operated at a speed of 150 rpm. After brushing for 1 min, the enamel blocks were rinsed with distilled water and soaked in SOF under the condition of a shaking water bath at 37°C and 60.0 rpm. After soaking for 3 to 4 hours, the enamel blocks were brushed with the samples by machine using the same procedure as in the first step. The brushing was repeated three times for one day, then the enamel blocks were kept in SOF overnight (>12 hours) in a shaking water bath at 37°C to mimic oral environment. After overnight incubation in SOF, the enamel blocks were brushed with water by machine using the same procedure as brushing with samples. These steps are considered as a whole treatment cycle.

### Results

For Samples 8 and 9, the enamel blocks were treated for three cycles then SEM images of the enamel blocks were taken. From corresponding cross-section SEM images, it appeared that a new layer was formed on the surface of the enamel blocks after treatment with Sample 9, while Sample 8 treated enamel blocks did not have a layer formed on their surfaces.

For Samples 10 and 11, the enamel blocks were treated for one, three, five and seven cycles. The changes in whiteness was measured as described in Example 2. The results of tooth whitening after treatment are summarized in table 8 (error represents standard deviation for duplicate measurements).

**TABLE 8**

| Change in WIO | Samples | |
|---|---|---|
| | **10** | **11** |
| One cycle | 1.73 ± 1.17 | 3.85 ± 2.43 |
| Three cycles | 3.50 ± 1.83 | 4.66 ± 2.65 |
| Five cycles^{d} | 4.24^{A} ± 2.73 | 10.80^{B} ± 2.20 |
| Seven cycles^{e} | 3.35^{A} ± 2.02 | 16.12^{B} ± 3.79 |

| | | |
|---|---|---|
| d. Values with different letter are significantly different (p<0.01). e. Values with different letter are significantly different (p<0.01). | | |

After five and seven cycles of treatment, Sample 11 comprising additional hydroxyapatite particles showed significantly better tooth whitening efficacy compared to Sample 10, which indicated a better deposition of whitening particles on tooth surfaces. After three cycles of treatments, SEM images were taken for both Samples 10 and 11, which demonstrated that a new layer was formed on the surface of the enamel blocks and TiO₂ particles were evenly embedded in the newly formed layer. Analysis using Energy Dispersive X-ray Spectroscopy (EDX) identified the elements of Ti, Ca, Zn and P within the new layer, indicating the deposition of TiO₂ particles on the tooth surfaces.

## Claims

1. An oral care composition comprising:
a) zinc oxide; and
b) calcium dihydrogen phosphate;
wherein the zinc oxide and the calcium dihydrogen phosphate are present in a weight ratio from 10:1 to 1:5.

2. The oral care composition as claimed in claim 1, wherein the zinc oxide is present in an amount of from 0.1 to 35% by weight of the composition, preferably from 0.5 to 20%.

3. The oral care composition as claimed in claim 1 or claim 2, wherein the calcium dihydrogen phosphate is present in an amount of from 0.01 to 20% by weight of the composition, preferably from 0.05 to 10%.

4. The oral care composition as claimed in any of the preceding claims, wherein the zinc oxide and the calcium dihydrogen phosphate is present in a weight ratio from 5:1 to 1:3, preferably from 3:1 to 1:2.

5. The oral care composition as claimed in any of the preceding claims, wherein the composition further comprises a benefit agent selected from optical agents, biomineralization agents, antibacterial agents, gum health agents, desensitizing agents, anti-calculus agents, freshness agents or mixtures thereof.

6. The oral care composition as claimed in claim 5, wherein the benefit agent is a biomineralization agent selected from one or more of fluoride sources, biomolecules, proteinaceous materials, amorphous calcium phosphate, α-tricalcium phosphate, β-tricalcium phosphate; calcium carbonate, calcium deficient hydroxyapatite Ca_{10-X}(HPO₄)_{X}(PO₄)_{6-X}(OH)_{2-X}, 0 ≤ x < 1), dicalcium phosphate (CaHPO₄), dicalcium phosphate dihydrate (CaHPO₄·2H₂O), hydroxyapatite (Ca₁₀(PO₄)₆(OH)₂), monocalcium phosphate monohydrate (Ca(H₂PO₄)₂·H₂O), octacalcium phosphate (Ca₈H₂(PO₄)₆·5H₂O) and tetracalcium phosphate (Ca₄(PO₄)₂O), preferably hydroxyapatite.

7. The oral care composition as claimed in claim 5, wherein the benefit agent is an optical agent, preferably a particulate whitening agent.

8. The oral care composition as claimed in claim 7, wherein the particulate whitening agent is a composite particle.

9. The oral care composition as claimed in claim 8, wherein the composite particle is titanium dioxide coated with calcium silicate.

10. The oral care composition as claimed in any one of claims 5 to 9, wherein the benefit agent comprises a combination of biomineralization agents and optical agents, preferably the biomineralization agent is hydroxyapatite and the optical agent is a particulate whitening agent.

11. The oral care composition as claimed in any one of claims 5 to 10, wherein the benefit agent is present in an amount of from 0.25 to 60%, preferably from 0.5 to 40% by weight of the composition.

12. The oral care composition as claimed in any one of claims 5 to 11, wherein the weight ratio of the zinc oxide to the benefit agent ranges from 1:30 to 20:1, preferably from 1:20 to 10:1.

13. The oral care composition as claimed in any of the preceding claims, wherein the oral care composition is a monophase anhydrous composition.

14. The oral care composition as claimed in any one of the preceding claims 1 to 12, wherein the composition is a dual-phase composition comprising a first phase and a second phase, wherein the zinc oxide is present in the first phase and the calcium dihydrogen phosphate is present in the second phase.

15. An oral care composition for use in a method for reducing sensitivity and/or remineralizing and/or whitening of the teeth of an individual comprising the step of applying the composition as claimed in any of the preceding claims to at least one surface of the teeth of the individual.

## Patentansprüche

1. Mundpflegezusammensetzung, umfassend:
a) Zinkoxid; und
b) Calciumdihydrogenphosphat;
wobei das Zinkoxid und das Calciumdihydrogenphosphat in einem Gewichtsverhältnis von 10:1 bis 1:5 vorliegen.

2. Mundpflegezusammensetzung wie in Anspruch 1 beansprucht, wobei das Zinkoxid in einer Menge von 0,1 bis 35 Gewichts-% der Zusammensetzung vorliegt, bevorzugt von 0,5 bis 20%.

3. Mundpflegezusammensetzung wie in Anspruch 1 oder Anspruch 2 beansprucht, wobei das Calciumdihydrogenphosphat in einer Menge von 0,01 bis 20 Gewichts-% der Zusammensetzung vorliegt, bevorzugt von 0,05 bis 10%.

4. Mundpflegezusammensetzung wie in irgendeinem der vorhergehenden Ansprüche beansprucht, wobei das Zinkoxid und das Calciumdihydrogenphosphat in einem Gewichtsverhältnis von 5:1 bis 1:3, bevorzugt von 3:1 bis 1:2, vorliegen.

5. Mundpflegezusammensetzung wie in irgendeinem der vorhergehenden Ansprüche beansprucht, wobei die Zusammensetzung ferner ein Vorteilsmittel umfasst, das ausgewählt ist aus optischen Mitteln, Biomineralisierungsmitteln, antibakteriellen Mitteln, Mitteln für die Zahnfleischgesundheit, Desensibilisierungsmitteln, Zahnsteinschutzmitteln, Frischhaltemitteln oder Mischungen davon.

6. Mundpflegezusammensetzung wie in Anspruch 5 beansprucht, wobei das Vorteilsmittel ein Biomineralisierungsmittel ist, das ausgewählt ist aus einer oder mehreren von Fluoridquellen, Biomolekülen, proteinhaltigen Materialien, amorphem Calciumphosphat, a-Tricalciumphosphat, β-Tricalciumphosphat; Calciumcarbonat, calciumarmem Hydroxyapatit (Ca₁₀₋ₓ(HPO₄)ₓ(PO₄)₆₋ₓ(OH)₂₋ₓ, 0 ≤ x < 1), Dicalciumphosphat (CaHPO₄), Dicalciumphosphatdihydrat (CaHPO₄·2H₂O), Hydroxyapatit (Ca₁₀(HPO₄)₆(OH)₂), Monocalciumphosphatmonohydrat (Ca(H₂PO₄)₂·H₂O), Octacalciumphosphat (Ca₈H₂(PO₄)₆·5H₂O) und Tetracalciumphosphat (Ca₄(PO₄)₂O), bevorzugt Hydroxyapatit.

7. Mundpflegezusammensetzung wie in Anspruch 5 beansprucht, wobei das Vorteilsmittel ein optisches Mittel ist, bevorzugt ein teilchenförmiges Weißungsmittel.

8. Mundpflegezusammensetzung wie in Anspruch 7 beansprucht, wobei das teilchenförmige Weißungsmittel ein Kompositteilchen ist.

9. Mundpflegezusammensetzung wie in Anspruch 8 beansprucht, wobei das Kompositteilchen Titandioxid ist, das mit Calciumsilicat beschichtet ist.

10. Mundpflegezusammensetzung wie in irgendeinem der Ansprüche 5 bis 9 beansprucht, wobei das Vorteilsmittel eine Kombination von Biomineralisierungsmitteln und optischen Mitteln umfasst, wobei es bevorzugt ist, dass das Biomineralisierungsmittel Hydroxyapatit ist und das optische Mittel ein teilchenförmiges Weißungsmittel ist.

11. Mundpflegezusammensetzung wie in irgendeinem der Ansprüche 5 bis 10 beansprucht, wobei das Vorteilsmittel in einer Menge von 0,25 bis 60 Gewichts-%, bevorzugt 0,5 bis 40 Gewichts-% der Zusammensetzung vorliegt.

12. Mundpflegezusammensetzung wie in irgendeinem der Ansprüche 5 bis 11 beansprucht, wobei das Gewichtsverhältnis von dem Zinkoxid zum Vorteilsmittel im Bereich von 1:30 bis 20:1 liegt, bevorzugt von 1:20 bis 10:1.

13. Mundpflegezusammensetzung wie in irgendeinem der vorhergehenden Ansprüche beansprucht, wobei die Mundpflegezusammensetzung eine einphasige, wasserfreie Zusammensetzung ist.

14. Mundpflegezusammensetzung wie in irgendeinem der vorhergehenden Ansprüche 1 bis 12 beansprucht, wobei die Zusammensetzung eine Zweiphasenzusammensetzung ist, die eine erste Phase und eine zweite Phase umfasst, wobei das Zinkoxid in der ersten Phase vorhanden ist und das Calciumdihydrogenphosphat in der zweiten Phase vorhanden ist.

15. Mundpflegezusammensetzung zur Verwendung in einem Verfahren zum Verringern der Empfindlichkeit und/oder zur Remineralisierung und/oder zum Aufhellen der Zähne eines Individuums, umfassend den Schritt des Aufbringens der Zusammensetzung, wie in irgendeinem der vorhergehenden Ansprüche beansprucht, auf mindestens eine Oberfläche der Zähne des Individuums.

## Revendications

1. Composition pour le soin oral comprenant :
a) de l'oxyde de zinc ; et
b) du dihydrogénophosphate de calcium ;
dans laquelle l'oxyde de zinc et le dihydrogénophosphate de calcium sont présents dans un rapport de masse de 10:1 à 1:5.

2. Composition pour le soin oral selon la revendication 1, dans laquelle l'oxyde de zinc est présent dans une quantité de 0,1 à 35 % en masse de la composition, de préférence de 0,5 à 20 %.

3. Composition pour le soin oral selon la revendication 1 ou revendication 2, dans laquelle le dihydrogénophosphate de calcium est présent dans une quantité de 0,01 à 20 % en masse de la composition, de préférence de 0,05 à 10 %.

4. Composition pour le soin oral selon l'une quelconque des revendications précédentes, dans laquelle l'oxyde de zinc et le dihydrogénophosphate de calcium sont présents dans un rapport de masse de 5:1 à 1:3, de préférence de 3:1 à 1:2.

5. Composition pour le soin oral selon l'une quelconque des revendications précédentes, dans laquelle la composition comprend de plus un agent bénéfique choisi parmi des agents optiques, agents de biominéralisation, agents antibactériens, agents pour la santé des gencives, agents de désensibilisation, agents anti-tartre, agents de fraicheur et mélanges de ceux-ci.

6. Composition pour le soin oral selon la revendication 5, dans laquelle l'agent bénéfique est un agent de biominéralisation choisi parmi une ou plusieurs sources de fluor, biomolécules, matériaux protéinés, phosphate de calcium amorphe, phosphate d'a-tricalcium, phosphate de β-tricalcium, carbonate de calcium, hydroxyapatite déficiente en calcium (Ca₁₀₋ₓ(HPO₄)ₓ(PO₄)₆₋ₓ(OH)₂₋ₓ, 0 ≤ x < 1), phosphate de dicalcium (CaHP04), phosphate de dicalcium dihydraté (CaHPO₄.2H₂O), hydroxyapatite (Ca₁₀(PO₄)₆(OH)₂), phosphate de monocalcium monohydraté (Ca(H₂PO₄)₂.H₂O), phosphate d'octacalcium (Ca₈H₂(PO₄)₆.5H₂O), et phosphate de tétracalcium (Ca₄(PO₄)₂O), de préférence hydroxyapatite.

7. Composition pour le soin oral selon la revendication 5, dans laquelle l'agent bénéfique est un agent optique, de préférence un agent blanchissant particulaire.

8. Composition pour le soin oral selon la revendication 7, dans laquelle l'agent blanchissant particulaire est une particule composite.

9. Composition pour le soin oral selon la revendication 8, dans laquelle la particule composite est du dioxyde de titane revêtu de silicate de calcium.

10. Composition pour le soin oral selon l'une quelconque des revendications 5 à 9, dans laquelle l'agent bénéfique comprend une combinaison d'agents de biominéralisation et d'agents optiques, de préférence l'agent de biominéralisation est l'hydroxyapatite et l'agent optique est un agent blanchissant particulaire.

11. Composition pour le soin oral selon l'une quelconque des revendications 5 à 10, dans laquelle l'agent bénéfique est présent dans une quantité de 0,25 à 60 %, de préférence de 0,5 à 40 % en masse de la composition.

12. Composition pour le soin oral selon l'une quelconque des revendications 5 à 11, dans laquelle le rapport de masse de l'oxyde de zinc à l'agent bénéfique est de 1:30 à 20:1, de préférence de 1:20 à 10:1.

13. Composition pour le soin oral selon l'une quelconque des revendications précédentes, dans laquelle la composition pour le soin oral est une composition anhydre monophase.

14. Composition pour le soin oral selon l'une quelconque des revendications 1 à 12 précédentes, dans laquelle la composition est une composition à phase double comprenant une première phase et une seconde phase, dans laquelle l'oxyde de zinc est présent dans la première phase et le dihydrogénophosphate de calcium est présent dans la seconde phase.

15. Composition pour le soin oral pour une utilisation dans un procédé pour la réduction de la sensibilité et/ou la reminéralisation et/ou le blanchiment des dents d'un individu comprenant l'étape d'application de la composition selon l'une quelconque des revendications précédentes sur au moins une surface des dents de l'individu.
